Europäisches Patentamt

(19) European Patent Office

Office européen des brevets

(11) Publication number: **0 104 198**
**B1**

(12) **EUROPEAN PATENT SPECIFICATION**

(45) Date of publication of patent specification: **19.02.86**

(21) Application number: **83900953.7**

(22) Date of filing: **24.03.83**

(86) International application number:
**PCT/GB83/00088**

(87) International publication number:
**WO 83/03347 13.10.83 Gazette 83/24**

(51) Int. Cl.⁴: **A 61 F 2/06**

(54) **VASCULAR PROSTHESIS.**

(30) Priority: **25.03.82 GB 8208852**

(43) Date of publication of application:
**04.04.84 Bulletin 84/14**

(45) Publication of the grant of the patent:
**19.02.86 Bulletin 86/08**

(84) Designated Contracting States:
**AT BE CH DE FR GB LI LU NL SE**

(56) References cited:
**FR-A-2 194 406**

(73) Proprietor: **J. & P. Coats, Limited**
**155 St. Vincent Street**
**Glasgow G2 5PA Scotland (GB)**

(72) Inventor: **HOOD, Robert Gordon**
**'Koinonia' 30 Park Avenue**
**Paisley Renfrewshire (GB)**

Courier Press, Leamington Spa, England.

## Description

The subject of this invention is a vascular prosthesis of the type comprising a porous tube made of textile material formed with circumferential corrugations intended to act as a substitute blood vessel in a human or an animal body.

In placing a vascular prosthesis in position in a human or animal body it is extremely desirable that the graft should not be twisted. To this end it is disclosed in FR—A—2 194 406 to provide on the graft at least one line of contrasting colour so that any twist in the graft becomes readily apparent. However, there is another consideration relating to the fitting of a graft. That is that the amount by which the graft is extended axially beyond its unstressed condition should be maintained within a predetermined limit. If a graft formed as a circumferential corrugated tube is not stretched far enough there may be an increased resistance presented to the flow of blood through the graft because of interference presented by the corrugations. If the graft is stretched too much the pores may be extended to a point where there is a danger of haemorrhage occurring at the junction between the graft and the host tissue before there has been time for the build up of tissue on the walls of the graft or the sutures at the junction may be pulled out of the host tissue by excessive stretch as a result of excessive tension in the graft itself.

The prior patent specification GB—A—1 374 627 discloses an elastic bandage carrying markings which change shape when the bandage is stretched to provide the desired amount of tension. The operating conditions of an elastic bandage are, however, quite different from the operating conditions of a vascular prosthesis. In use of an elastic bandage the bandage must be applied under tension otherwise it has no effect. In a vascular prosthesis which is necessarily porous the pore size is critical and as is explained above it must be maintained with little or no change from the pore size provided during manufacture. Thus the material of the prosthesis must not on any account be stretched and any markings provided must be such that when the prosthesis is extended to the correct amount the change in the appearance of the markings must be very clearly evident to the surgeon making the implant. The form of marking which is acceptable in the elastic bandage of specification GB—A—1 374 627 would not provide a signal which the user could not mistake. For example, there is probably insufficient difference between oblongs and squares, or diamonds which become wider diamonds, or equally spaced lines the spacings of which is simply increased as stretching of the bandage takes place. Such changes of degree are to many people not readily apparent at all and in any event require a recollection of what the markings were like before the bandage was extended. Such markings would thus not be effective on a vascular prosthesis and might even lead to confusion.

It is an object of the present invention to provide an indicating means on a circumferentially corrugated graft which provides a changing appearance based on a change in the original appearance of the markings when the graft is extended to the correct extent which does not require the exercise of memory by the surgeon implating the graft, i.e. he does not require to try to remember what the marking looked like before he extended the graft because the extended graft shows a marking with a difference which is absolute in the sense that that marking will appear only in an unique form when the correct amount of extension of the graft has been attained.

According to the invention a vascular prosthesis which comprises a circumferentially corrugated tube of textile material is characterized by presenting a pattern of a series of separate axially spaced markings which are applied as regularly shaped and spaced markings to the surface of the uncorrugated tube before it was corrugated so that after corrugation of the tube the pattern of these markings shows irregularities or is unsymmetrical when the unextended tube is viewed from the side as a result of the varying inclination of the corrugated surface with respect to the axis of the prosthesis but when the prosthesis is extended to the correct amount of extension in which the corrugations are almost straightened the pattern of the markings revert substantially to its original form free from any irregularity or dissymmetry whereby to indicate that no stretching of the material of the extended prosthesis has taken place.

In one construction, on each crest and/or in each trough, there is provided a short axial line so disposed that before the prosthesis is extended the lines are aligned longitudinally but all the gaps between the lines in a longitudinal direction are of a length different from the length of the lines and when the prosthesis is extended to the correct extent the lengths of the gaps appear to be the same as the lengths of the individual lines thus providing a dashed line from one end of the prosthesis to the other with the lengths of lines and spaces all equal.

Alternatively the prosthesis may be marked with spaced circumferential lines each of which forms a short arc on the surface of the prosthesis, the axial positioning and spacing of the lines being such that before the prosthesis is extended the gaps between pairs of adjacent lines are all of different width but when the prosthesis is extended by the correct amount the arcs when viewed from one side of the prosthesis are spaced equidistantly from one another, i.e. the arcs are so positioned that when the prosthesis is in the unstressed state the arcs appear in groups of closely spaced lines and when the prosthesis is extended to the correct extent the radially projected lengths of the gaps between all the arcs become equal to an observer.

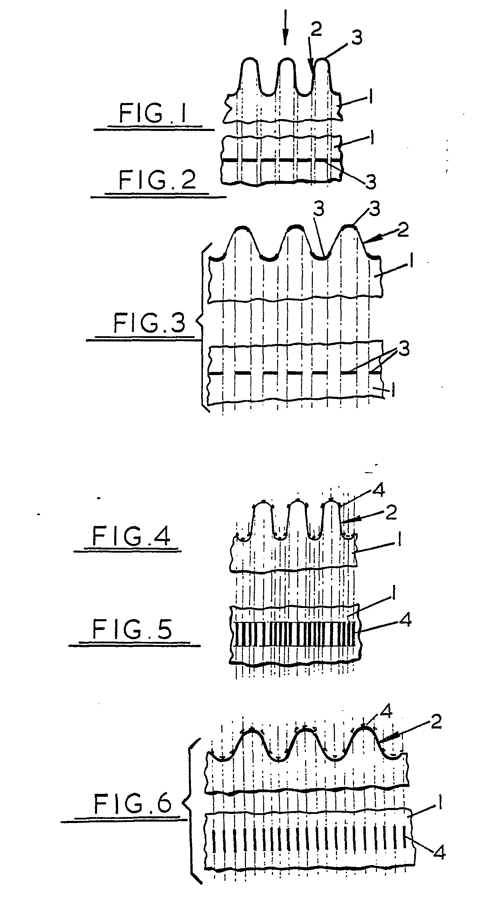

Practical embodiments of the invention are illustrated in the accompanying drawings in which Fig. 1 illustrates a vascular prosthesis in the unstressed condition indicating in side elevation the manner in which the individual axial lines are applied to the prosthesis, Fig. 2 shows how the prosthesis appears in the unextended condition looking down into the corrugations and showing the appearance of the markings and Fig. 3 shows the prosthesis of Figs. 1 and 2 extended to the correct extent indicating how the markings then appear, Fig. 4 illustrates a prosthesis with another form of marking in the form of arcs orientated circumferentially on the prosthesis, Fig. 5 illustrates how the prosthesis of Fig. 4 appears when looked at in the unextended condition and Fig. 6 illustrates the prosthesis of Figs. 4 and 5 extended to the correct extent showing the markings clearly indicating that this situation has been reached by the equal spacing of the markings.

In the drawings 1 denotes the prosthesis formed with corrugations 2. In Figs. 1, 2 and 3, the numeral 3 denotes axially orientated lines marked on the tube constituting the prosthesis while in Figs. 4, 5 and 6, the numeral 4 denotes arcuate lines marked on the tube constituting the prosthesis.

Referring first to the construction of Figs. 1, 2 and 3, the prosthesis appears as illustrated in Fig. 2 with the lines marked thereon indicating a more or less continuous line or at least with gaps between adjacent lines much smaller than the length of the lines themselves. When the prosthesis is extended to the correct extent for use as a graft the angle between the sides of each corrugation is increased so that the radially projected length of each line or each space as the case may be as seen by an observer is changed to show a dashed line along the length of the prosthesis in which the individual lines and spaces are of equal length.

In the construction of Figs. 4, 5 and 6, the unextended prosthesis appears as in Fig. 5 with the lines appearing in groups in which the spacing is irregular. When the prosthesis is extended to the desirable extent for use as a graft once again the projected distance between adjacent lines is changed because of the change in angularity of the surface carrying the lines and they appear all equi-spaced in an axial direction.

A surgeon extending a prosthesis according to the invention in making an implant does not have to remember what the markings were like before the prosthesis was extended. When the markings are all equally spaced or the gaps between individual markings have the same length as the markings themselves he knows that the prosthesis has been extended by the correct amount without stretch in the material of the prosthesis.

## Claims

1. A vascular prosthesis which comprises a circumferentially corrugated tube (1) of textile material is characterized by presenting a series of separate axially spaced markings (3, 4) which are applied as regularly shaped and spaced markings to the surface of the uncorrugated tube before it was corrugated so that after corrugation of the tube the pattern of these markings shows irregularities or is unsymmetrical when the unextended tube is viewed from the side as a result of the varying inclination of the corrugated surface with respect to the axis of the prosthesis but when the prosthesis is extended to the correct amount of extension in which the corrugations ars almost straightened the pattern of the markings reverts substantially to its original form free from any irregularity or dissymmetry whereby to indicate that no stretching of the material of the extended prosthesis has taken place.

2. A vascular prosthesis according to claim 1, characterized in that on each crest and/or in each trough there is provided a short axial line (3) so disposed that before the prosthesis is extended the lines are aligned longitudinally but all the gaps between the lines in a longitudinal direction are of a length different from the length of the lines and when the prosthesis is extended to the correct extent the lengths of the gaps appear to be the same as the lengths of the individual lines thus providing a dashed line from one end of the prosthesis to the other with the lengths of lines and spaces all equal.

3. A vascular prosthesis according to claim 1 characterized in that the prosthesis is marked with spaced circumferential lines (4) each of which forms a short arc on the surface of the prosthesis, the axial positioning and spacing of the lines being such that before the prosthesis is extended the gaps between pairs of adjacent lines are all of different width but when the prosthesis is extended by the correct amount the arcs when viewed from one side of the prosthesis are spaced equi-distantly from one another, i.e. the arcs are so positioned that when the prosthesis is in the unstressed state the arcs appear in groups of closely spaced lines and when the prosthesis is extended to the correct extent the radially projected lengths of the gaps between all the arcs become to the eye of an observer equal.

### Revendications

1. Prothèse vasculaire comprenant un tube ondulé circonférentiellement (1) en matière textile, caractérisée par le fait qu'elle présente une série de marques (3, 4) séparées, espacées axialement, qui sont faites sous forme de marques de forme et d'espacement réguliers à la surface du tube avant que celui-ci ne soit ondulé, de façon qu'après l'ondulation du tube, le dessin de ces marques présente des irrégularités ou soit asymétrique lorsque le tube non étiré est regardé de côté, par suite de l'inclinaison variable de la surface ondulée par rapport à l'axe de la prothèse, mais lorsque la prothèse est étirée de la quantité correcte, pour laquelle les ondulations sont à peu près redressées, le dessin des marques reprenne sensiblement sa forme originale sans aucune irrégularité ou dissymétrie, pour indiquer ainsi qu'aucune tension de la matière de la prothèse étirée n'a au lieu.

2. Prothèse vasculaire selon la revendication 1, caractérisée par le fait que sur chaque crête et/ou dans chaque creux est fait un court trait axial (3) placé de façon qu'avant que la prothèse ne soit étirée, les traits soient alignés longitudinalement, tous les intervalles entre les traits dans la direction longitudinale étant cependant de longueur différente de celle des traits, et, lorsque la prothèse est étirée de la quantité correcte, la longueur des intervalles apparaisse égale à celle des traits, produisant ainsi une ligne en tirets d'une extrémité à l'autre de la prothèse, les longueurs des traits et des intervalles étant toutes égales.

3. Prothèse vasculaire selon la revendication 1, caractérisée par le fait qu'elle porte des traits circonférentiels espacés (4) formant chacun un arc court à sa surface, la position et l'espacement axiaux de ces traits étant tels qu'avant que la prothèse ne soit étirés, les intervalles entre traits voisins soient tous de longueur différente, mais lorsque la prothèse est étirée de la quantité correcte, les arcs, vus depuis un côté de la prothèse, soient équidistants, c'est-à-dire que les arcs sont placés de façon que lorsque le prothèse n'est pas étirée, ils apparaissent sous la forme de groupes de traits rapprochés, et lorsque la prothèse est étirée de la quantité correcte, les longueurs, projetées radialement, des intervalles entre tous les arcs deviennent égales pour l'oeil d'un observateur.

## Patentansprüche

1. Künstliches Blutgefäss aus einem wellig quergerippten Schlauch (1) aus textilem Material, dadurch gekennzeichnet, dass dieses ein Muster einer Reihe von axial getrennten Markierungen (3, 4) aufweist, welche gleichmässig geformt und mit gleichen Abständen auf die Oberfläche des noch nicht wellig quergerippten Schlauchs aufgebracht werden, so dass nach dem welligen Querrippen des Schlaucs das Muster dieser Markierungen als Folge der, bezogen auf die Achse des künstlichen Blutgefässes ändernden Neigung der welligen Querrippen, Ungleichmässigkeit aufweist oder asymmetrisch ist, wenn der nicht gestreckte Schlauch von der Seite betrachtet wird, dass aber das Muster der Markierungen im wesentlichen die ursprüngliche Form, frei von Unregelmässigkeiten oder Asymmetrien annimmt, wenn das künstliche Blutgefäss auf die richtige Länge gestreckt ist, bei welcher die welligen Querrippen beinahe ausgestreckt sind, und womit angezeigt wird, dass kein Dehnen des Materials des gestreckten künstlichen Blutgefässes aufgetreten ist.

2. Künstliches Blutgefäss nach Patentanspruch 1, dadurch gekennzeichnet, dass auf jedem Wellenkamm und/oder in jedem Wellental eine kurze axiale Linie (3) derart angeordnet ist, dass, vor dem Strecken des künstlichen Blutgefässes die Linien längs hintereinander gereiht sind, dass aber die Zwischenräume zwischen den Linien in Längsrichtung, von der Länge der einzelnen Linien verschieden sind, und dass die Linien und die Zwischenräume zwischen den Linien gleich lang erscheinen, wenn das künstliche Blutgefäss auf die richtige Länge gestreckt ist, sodass das künstliche Blutgefässe, von einem Ende zum andern, eine gestrichelte Linie mit Strichen und Zwischenräumen gleicher Länge aufweist.

3. Künstliches Blutgefäss nach Patentanspruch 1, dadurch gekennzeichnet, dass das künstliche Blutgefäss auf seinem Umfang Querlinien (4) mit Zwischenräumen aufweist, von denen jede auf der Oberfläche des künstlichen Blutgefässes einen kurzen Bogen bildet wobei die axiale Anordnung und der Zwischenraum derart gewählt sind, dass, die Abstände zwischen Paaren von benachbarten Linien verschieden sind, wenn das künstliche Blutgefäss ausgestreckt ist, aber alle Abstände zwischen den Bogen gleich gross sind, wenn das künstliche Blutgefäss auf die richtige Länge gestreckt ist und von der Seite betrachtet wird, d.h. die Bogen sind so angeordnet, dass sie bei entspanntem Zustand des künstlichen Blutgefässes, von der Seite betrachtet als Gruppen nahe beieinanderliegender Linien erscheinen und dass die radial projizierte Länge der Zwischenräume zwischen allen Bogen dem Auge eines Beobachters gleichlang erscheint, wenn das künstliche Blutgefäss um die richtige Länge gestreckt wurde.

FIG.1

FIG.2

FIG.3

FIG.4

FIG.5

FIG.6